# EUROPÄISCHE PATENTSCHRIFT

(11) **EP 1 255 525 B1**
(45) Veröffentlichungstag und Bekanntmachung des Hinweises auf die Patenterteilung: **29.10.2003**
(21) Anmeldenummer: 01936050.2
(22) Anmeldetag: 08.02.2001
(51) Int. Cl.: A61K 7/00

(54) **KOSMETISCHE ZUBEREITUNGEN IN FORM DISPERSER SYSTEME PERLGLANZWACHSE ENTHALTEND**
PEARLY LUSTRE MEANS
PRODUIT A LUSTRE NACRE

(30) Priorität: 17.02.2000 DE 10007322
(43) Veröffentlichungstag der Anmeldung: 13.11.2002
(73) Patentinhaber: Cognis Deutschland GmbH & Co. KG, 40589 Düsseldorf (DE)
(72) Erfinder: NIEENDICK, Claus, 47807 Krefeld (DE); SCHMID, Karl, Heinz, 40822 Mettmann (DE); EGGERS, Anke, 40215 Düsseldorf (DE)
(86) Internationale Anmeldenummer: EP0101349
(87) Internationale Veröffentlichungsnummer: WO01060323

(56) Entgegenhaltungen:
- EP-A- 0 974 332
- WO-A-94/28867
- WO-A-95/33817
- DE-A- 4 336 407
- DATABASE WPI Section Ch, Week 199621 Derwent Publications Ltd., London, GB; Class D16, AN 1996-205431 XP002178431 & JP 08 073344 A (KANEBO LTD), 19. März 1996 (1996-03-19)
- PATENT ABSTRACTS OF JAPAN vol. 1998, no. 01, 30. Januar 1998 (1998-01-30) & JP 09 249529 A (KANEBO LTD), 22. September 1997 (1997-09-22)
- PATENT ABSTRACTS OF JAPAN vol. 014, no. 242 (C-0721), 23. Mai 1990 (1990-05-23) & JP 02 062817 A (NIKKO KEMIKARUZU KK), 2. März 1990 (1990-03-02)

## Beschreibung

### Gebiet der Erfindung

Die Erfindung beschreibt Zubereitungen, die in einer kontinuierlichen, zusammenhängenden Phase dispergierte Wirkstoffe enthalten.

### Stand der Technik

Zubereitungen, die in der Kosmetik Anwendung finden, enthalten neben den üblichen kosmetischen Inhaltsstoffen Wirkstoffe, wie beispielsweise biogene Wirkstoffe, Vitamine, Deodorantien, Antischuppenmittel, UV-Lichtschutzfaktoren und dergleichen. Wünschenswert ist die Beständigkeit der Wirkstoffe in kosmetischen Formulierungen durch andere Anbietungsformen zu verbessern. Die Europäische Patentschrift **EP 0764201 B1** beschreibt beispielsweise Reinigungsmittel, welche Peroxidverbindungen als Wirkstoffe verkapselt in Paraffinwachsen enthalten.

DE-A-43 36 407 und EP-A-0 974 332 beschreiben kosmetische Zusammensetzungen bestehend aus einer kontinuierlichen Phase aus Wachsen u.a. tierische Wachse, Fettsäuren, Ester oder Amide von Fettsäuren und Fettalkohole.

Die Aufgabe der vorliegenden Erfindung hat somit darin bestanden, Mittel zur Verfügung zu stellen, die es erlauben, eine hohe Konzentration von Wirkstoffen mittels eines Trägermaterials in kosmetischen Zubereitungen einzuarbeiten und dadurch die Stabilität der Wirkstoffe zu verbessern. Diese Mittel sollen gewährleisten, dass die Freisetzung der Wirkstoffe erst bei der Anwendung infolge mechanischer, thermischer, chemischer oder enzymatischer Einwirkung erfolgt und dadurch deren Wirkungsdauer verlängert und somit verbessert wird (z.B. in Haarkuren). Weiterhin soll die Beständigkeit von leicht instabilen Wirkstoffen in diesen Mitteln erhöht sein.

### Beschreibung der Erfindung

Gegenstand der Erfindung sind kosmetische Zubereitungen bestehend aus einer kontinuierlichen Phase und darin dispergierten Wirkstoffen, welche sich dadurch auszeichnen, dass die kontinuierliche Phase aus Perlglanzwachsen besteht.

Überraschenderweise wurde gefunden, daß sich Wirkstoffe, welche in einem Perlglanzwachs kontinuierlich dispergiert sind, hervorragend in kosmetische Zubereitungen einarbeiten lassen. Da die Wirkstoffe gewissermaßen vom Perlglanzwachs ganz oder teilweise umhüllt sind, ist eine Dosierung der Wirkstoffe über das Trägermaterial, d.h. das Perlglanzwachs möglich und erlaubt die Einarbeitung höherer Konzentrationen an Wirkstoffen in kosmetischen Zubereitungen. Die Freisetzung der Wirkstoffe wird durch die Perlglanzwachse verlangsamt und erfolgt ggf. erst durch mechanische, thermische, chemische oder enzymatische Einwirkung. Vorteilhaft ist hierbei ebenfalls, dass die Beständigkeit von Wirkstoffen, die beispielsweise leicht instabil sind, erhöht wird und deren Wirksamkeit bei der Anwendung durch verzögerte Freisetzung verbessert werden kann (z.B. Haarkuren u.a.).

Weitere Gegenstände der Erfindung betreffen zwei Verfahren zur Herstellung von kosmetischen und/oder pharmazeutischen Zubereitungen bei dem man Periglanzwachse und Wirkstoffe einer gemeinsamen oder getrennten Sprühtrocknung oder Sprühkristallisation unterwirft.

### Perlglanzwachse

Als Perlglanzwachse kommen beispielsweise in Frage: Alkylenglycolester; Fettsäurealkanolamide; Partialglyceride; Ester von mehrwertigen, gegebenenfalls hydroxysubstituierten Carbonsäuren mit Fettalkoholen mit 6 bis 22 Kohlenstoffatomen; Fettstoffe, wie beispielsweise Fettalkohole, Fettketone, Fettaldehyde, Fettether und Fettcarbonate, die in Summe mindestens 24 Kohlenstoffatome aufweisen; Ringöffnungsprodukte von Olefinepoxiden mit 12 bis 22 Kohlenstoffatomen mit Fettalkoholen mit 12 bis 22 Kohlenstoffatomen, Fettsäuren und/oder Polyolen mit 2 bis 15 Kohlenstoffatomen und 2 bis 10 Hydroxylgruppen sowie deren Mischungen. In einer bevorzugten Ausführungsform der Erfindung können die Perlglanzwachse fest oder flüssig sein.
- **Alkylenglycolester.** Bei den Alkylenglycolestern handelt es sich üblicherweise um Mono- undloder Diester von Alkylenglycolen, die der Formel **(I)** folgen,

   **R**^{**1**}**CO(OA)**_{**q**}**OR**^{**2**} **(I)**

   in der R¹CO für einen linearen oder verzweigten, gesättigten oder ungesättigten Acylrest mit 6 bis 22 Kohlenstoffatomen, R² für Wasserstoff oder R¹CO und A für einen linearen oder verzweigten Alkylenrest mit 2 bis 4 Kohlenstoffatomen und q für Zahlen von 1 bis 5 steht. Typische Beispiele sind Mono- und/oder Diester von Ethylenglycol, Propylenglycol, Diethylenglycol, Dipropylenglycol, Triethylenglycol oder Tetraethylenglycol mit Fettsäuren mit 6 bis 22, vorzugsweise 12 bis 18 Kohlenstoffatomen als da sind: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Ethylenglycolmono- und/oder -distearat.
- **Fettsäurealkanolamide.** Fettsäurealkanolamide, die als Perlglanzwachse in Frage kommen, folgen der Formel **(II)**,

   **R**^{**3**}**CO-NR**^{**4**}**-B-OH (II)**

   in der R³CO für einen linearen oder verzweigten, gesättigten oder ungesättigten Acylrest mit 6 bis 22 Kohlenstoffatomen, R⁴ für Wasserstoff oder einen gegebenenfalls hydroxysubstituierten Alkylrest mit 1 bis 4 Kohlenstoffatomen und B für eine lineare oder verzweigte Alkylengruppe mit 1 bis 4 Kohlenstoffatomen steht. Typische Beispiele sind Kondensationsprodukte von Ethanolamin, Methylethanolamin, Diethanolamin, Propanolamin, Methylpropanolamin und Dipropanolamin sowie deren Mischungen mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Stearinsäureethanolamid.
- **Partialglyceride.** Partialglyceride, die über Perlglanzeigenschaften verfügen, stellen Monound/oder Diester des Glycerins mit Fettsäuren, nämlich beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen dar. Sie folgen der Formel **(III)**, 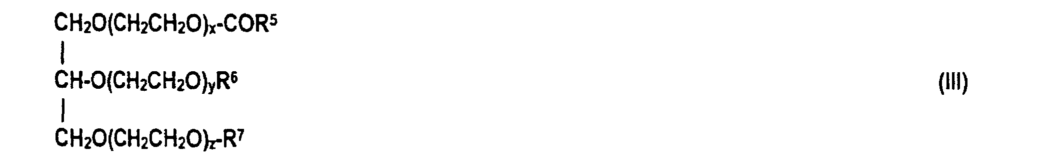 in der R⁵CO für einen linearen oder verzweigten Acylrest mit 6 bis 22 Kohlenstoffatomen, R⁵ und R⁷ unabhängig voneinander für Wasserstoff oder R⁷CO, x, y und z in Summe für 0 oder für Zahlen von 1 bis 30 und X für ein Alkali- oder Erdalkalimetall mit der Maßgabe steht, daß mindestens einer der beiden Reste R⁶ und R⁷ Wasserstoff darstellt. Typische Beispiele sind Laurinsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Isostearinsäuremonoglycend, Isostearinsäurediglycerid, Ölsäuremonoglycerid, Ölsäurediglycerid, Talgfettsäuremonoglycerid, Talgfettsäurediglycerid, Behensäuremonoglycerid, Behensäurediglycerid, Erucasäuremonoglycerid, Erucasäurediglycerid sowie deren technische Gemische, die untergeordnet aus dem Herstellungsprozeß noch geringe Mengen an Triglycerid enthalten können.
- **Mehrwertige Carbonsäure- und Hydroxycarbonsäureester.** Als Perlglanzwachse kommen weiterhin Ester von mehrwertigen, gegebenenfalls hydroxysubstituierten Carbonsäuren mit Fettalkoholen mit 6 bis 22 Kohlenstoffatomen in Frage. Als Säurekomponente dieser Ester kommen beispielsweise Malonsäure, Maleinsäure, Fumarsäure, Adipinsäure, Sebacinsäure, Azelainsäure, Dodecandisäure, Phthalsäure, Isophthalsäure und insbesondere Bemsteinsäure sowie Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Ester können als Voll- oder Partialester vorliegen, vorzugsweise werden Mono- und vor allem Diester der Carbon- bzw. Hydroxycarbonsäuren eingesetzt. Typische Beispiele sind Bemsteinsäuremono- und -dilaurylester, Bemsteinsäuremono- und -dicetearlyester, Bemsteinsäuremono- und -distearylester, Weinsäuremono- und -dilaurylester, Weinsäuremono- und dikokosalkylester, Weinsäuremono- und -dicetearylester, Citronensäuremono-, -di- und -trilaurylester, Citronensäuremono-, -di- und -trikokosalkylester sowie Citronensäuremono-, -di- und -tricetearylester.
- **Fettalkohole.** Als weitere Gruppe von Perlglanzwachsen können langkettige Fettalkohole eingesetzt werden, die der Formel **(IV)** folgen,

   **R**^{**8**}**OH (IV)**

   in der R⁸ für einen linearen Alkylrest mit 24 bis 48, vorzugsweise 32 bis 36 Kohlenstoffatomen steht. Bei den genannten Stoffen handelt es sich in der Regel um Oxidationsprodukte langkettiger Paraffine.
- **Fettketone.** Fettketone, die als Komponente (a) in Betracht kommen, folgen vorzugsweise der Formel **(V)**,

   **R**^{**9**}**-CO-R**^{**10**} **(V)**

   in der R⁹ und R¹⁰ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Ketone können nach Verfahren des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste R⁹ und R¹⁰ aber nur um ein Kohlenstoffatom und leiten sich von Fettsäuren mit 16 bis 22 Kohlenstoffatomen ab. Dabei zeichnet sich Stearon durch besonders vorteilhafte Perlglanzeigenschaften aus.
- **Fettaldehyde.** Als Perlglanzwachse geeignete Fettaldehyde entsprechen der Formel **(VI),**

   **R**^{**11**}**COH (VI)**

   in der R¹¹CO für einen linearen oder verzweigten Acylrest mit 24 bis 48, vorzugsweise 28 bis 32 Kohlenstoffatomen steht.
- **Fettether.** Als Perlglanzwachse kommen femer Fettether der Formel **(VII)** in Frage,

   **R**^{**12**}**-O-R**^{**13**} **(VII)**

   in der R¹² und R¹³ unabhängig voneinander für Alkyt- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Fettether der genannten Art werden üblicherweise durch saure Kondensation der entsprechenden Fettalkohole hergestellt. Fettether mit besonders vorteilhaften Perlglanzeigenschaften werden durch Kondensation von Fettalkoholen mit 16 bis 22 Kohlenstoffatomen, wie beispielsweise Cetylalkohol, Cetearylalkohol, Stearylalkohol, Isostearylalkohol, Oleylalkohol, Behenylalkohol und/oder Erucylalkohol erhalten.
- **Fettcarbonate.** Als Komponente (a) kommen weiterhin Fettcarbonate der Formel **(VIII)** in Betracht,

   **R**^{**14**}**O-CO-OR**^{**15**} **(VIII)** (VIII)

   in der R¹⁴ und R¹⁵ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Stoffe werden erhalten, indem man beispielsweise Dimethyloder Diethylcarbonat mit den entsprechenden Fettalkoholen in an sich bekannter Weise umestert. Demzufolge können die Fettcarbonate symmetrisch oder unsymmetrisch aufgebaut sein. Vorzugsweise werden jedoch Carbonate eingesetzt, in denen R¹⁴ und R¹⁵ gleich sind und für Alkylreste mit 16 bis 22 Kohlenstoffatomen stehen. Besonders bevorzugt sind Umesterungsprodukte von Dimethyl- bzw. Diethylcarbonat mit Cetylalkohol, Cetearylalkohol, Stearylalkohol, Isostearylalkohol, Oleylalkohol, Behenylalkohol und/ oder Erucylalkohol in Form ihrer Mono- und Diester bzw. deren technischen Mischungen.
- **Fettsäuren.** Für diesen Zweck kommen aliphatische, gegebenenfalls hydroxysubstituierte Carbonsäuren mit 16 bis 30 Kohlenstoffen in Frage, wie beispielsweise Stearinsäure, Cetylstearinsäure, Hydroxystearinsäure und Behensäure sowie deren technische Gemische.
- **Epoxidringöffnungsprodukte.** Bei den Ringöffnungsprodukten handelt es sich um bekannte Stoffe, die üblicherweise durch säurekatalysierte Umsetzung von endständigen oder innenständigen Olefinepoxiden mit aliphatischen Alkoholen hergestellt werden. Die Reaktionsprodukte folgen vorzugsweise der Formel **(IX)**, 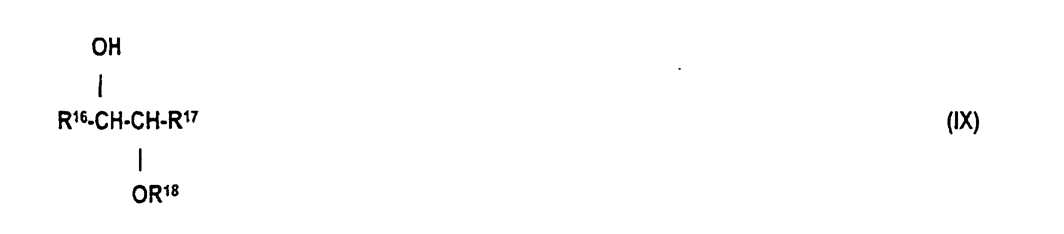 in der R¹⁶ und R¹⁷ für Wasserstoff oder einen Alkylrest mit 10 bis 20 Kohlenstoffatomen steht, mit der Maßgabe, daß die Summe der Kohlenstoffatome von R¹⁶ und R¹⁷ im Bereich von 10 bis 20 liegt und R¹⁸ für einen Alkyl- und/oder Alkenylrest mit 12 bis 22 Kohlenstoffatomen und/oder den Rest eines Polyols mit 2 bis 15 Kohlenstoffatomen und 2 bis 10 Hydroxylgruppen steht. Typische Beispiele sind Ringöffnungsprodukte von α-Dodecenepoxid, α-Hexadecenepoxid, α-Octadecenepoxid, α-Eicosenepoxid, α-Docosenepoxid, i-Dodecenepoxid, i-Hexadecenepoxid, i-Octadecenepoxid, i-Eicosenepoxid und/oder i-Docosenepoxid mit Laurylalkohol, Kokosfettalkohol, Myristylalkohol, Cetylalkohol, Cetearyl-alkohol, Stearylalkohol, Isostearylalkohol, Oleylalkohol, Elaidylalkohol, Petroselinylalkohol, Linolylalko-hol, Linolenylalkohol, Behenylalkohol und/oder Erucylalkohol. Vorzugsweise werden Ringöffnungsprodukte von Hexa- und/oder Octadecenepoxiden mit Fettalkoholen mit 16 bis 18 Kohlenstoffatomen eingesetzt. Werden anstelle der Fettalkohole Polyole für die Ringöffnung eingesetzt, so handelt es sich beispielsweise um folgende Stoffe: Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylofpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin. Die erfindungsgemäßen Zubereitungen können die Perlglanzwachse in Mengen von 50 bis 99,9, vorzugsweise 55 bis 90 und insbesondere 60 bis 85 Gew.-% - bezogen auf die Zubereitungen - enthalten.

### Wirkstoffe für kosmetische und/oder pharmazeutische Anwendungen

Typische Beispiele für Wirkstoffe, wie sie im Bereich der kosmetischen und pharmazeutischen Zubereitungen eingesetzt werden sind Tenside, kosmetische Öle, Stabilisatoren, biogene Wirkstoffe, Vitamine, Deodorantien, Antitranspirantien, Antischuppenmittel, UV-Lichtschutzfaktoren, Antioxidantien, Konservierungsmittel, Insektenrepellentien, Selbstbräuner, Typrosininhibitoren (Depigmentierungsmittel), Parfümöle und Farbstoffe. Diese Wirkstoffe können in Mengen von 0,1 bis 50, vorzugsweise 10 bis 45 und insbesondere 15 bis 40 Gew.-% - bezogen auf die Zubereitungen - enthalten sein.

Als oberflächenaktive Stoffe können anionische, nichtionische, kationische und/oder amphotere bzw. amphotere Tenside verkapselt werden. Typische Beispiele für **anionische Tenside** sind Seifen, Alkylbenzolsulfonate, Aikansulfonate, Olefinsulfonate, Alkylethersutfonate, Glycerinethersulfonate, α-Methytestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag. Berlin, 1987, S. 54-124 oder J.Falbe (ed.), "Katalysatoren, Tenside und Mineralöladditive", Thieme Verlag, Stuttgart, 1978, S. 123-217** verwiesen.

Als **kosmetische Öle** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Kojisäure, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder-phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
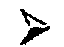 adstringierende Wirkstoffe,
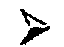 Ölkomponenten,
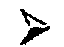 nichtionische Emulgatoren,
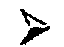 Coemulgatoren,
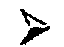 Konsistenzgeber,
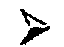 Hilfsstoffe wie z. B. Verdicker oder Komplexierungsmittel und/oder
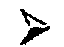 nichtwässrige Lösungsmittel wie z. B. Ethanol, Propylenglykol und/oder Glycerin

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

Daneben können in Antitranspirantien übliche öllösliche und wasserlösliche Hilfsmittel in geringeren Mengen enthalten sein. Solche öllöslichen Hilfsmittel können z.B. sein:
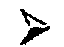 entzündungshemmende, hautschützende oder wohlriechende ätherische Öle,
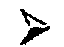 synthetische hautschützende Wirkstoffe und/oder
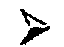 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Climbazol, Octopirox, Ketokonazol und Zinkpyrethion eingesetzt werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UV-B-Filter können öllöslich oder wasserlöslich sein. Als öllösliche Substanzen sind z.B. zu nennen:
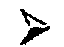 3-Benzylidencampher bzw. 3-Benzylidennorcampher und dessen Derivate, z.B. 3-(4-Methylbenzyliden)campher wie in der **EP 0693471 B1** beschrieben;
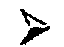 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
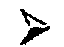 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
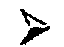 Ester der Salicylsäure, vorzugsweise Salicylsäure-2-ethylhexylester, Salicylsäure-4-isopropylbenzylester, Salicylsäurehomomenthylester;
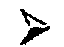 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
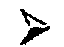 Ester der Benzalmalonsäure, vorzugsweise 4-Methoxybenzmalonsäuredi-2-ethylhexylester;
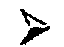 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
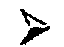 Propan-1,3-dione, wie z.B. 1-(4-tert Butylphenyl)-3-(4'methoxyphenyl)propan-1,3-dion;
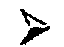 Ketotricyclo(5.2.10)decan-Derivate, wie in der **EP 0694521 B1** beschrieben.

Als wasserlösliche Substanzen kommen in Frage:
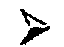 2-Phenylbenzimidazol-5-sulfonsäure und deren Alkali-, Erdalkali-, Ammonium-, Alkylammonium-, Alkanolammonium- und Glucammoniumsalze;
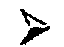 Sulfonsäurederivate von Benzophenonen, vorzugsweise 2-Hydroxy-4-methoxybenzophenon-5-sulfonsäure und ihre Salze;
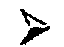 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikrooder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyt-m-toluamid, 1,2-Pentandiol oder Ethyl Butylacetylaminopropionate in Frage, als **Selbstbräuner** eignet sich Dihydroxyaceton. Als **Tyrosinhinbitoren**, die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilliat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Wirkstoffe können auch ausschließlich aus ästhetischen Gründen in den Kapseln enthalten und nicht für eine kontrollierte Freigabe vorgesehen sein.

### Sprühtrocknung oder Sprühkristallisation

Bei der Trockeneinrichtung, in die die Perigfanzwachse und Wirkstoffe versprüht werden, kann es sich um beliebige Trockenapparaturen handeln. In einer bevorzugten Verfahrensführung wird die Trocknung als Sprühtrocknung bzw. Sprühkristallisation in einem Trockenturm und besonders bevorzugt unter vermindertem Druck. Dabei werden die wäßrigen Zubereitungen in bekannter Weise einem Trocknungsgasstrom in feinverteilter Form ausgesetzt. In Patentveröffentlichungen der Firma Cognis wird eine Ausführungsform der Sprühtrocknung mit überhitztem Wasserdampf beschrieben. Verwiesen wird hier insbesondere auf die nachfolgenden Druckschriften: **DE 4030688 A1** sowie die weiterführenden Veröffentlichungen gemäß **DE 4204035 A1; DE 4204090 A1; DE 4206050 A1; DE 4206521 A1; DE 4206495 A1; DE 4208773 A1; DE 4209432 A1** und **DE 4234376 A1.** Dieses Verfahren wurde schon im Zusammenhang mit der Herstellung des Entschäumerkom vorgestellt.

Die Perlglanzwachse und Wirkstoffe können gemeinsamen Sprühtrocknung oder Sprühkristallisation unterworfen werden. Weiterhin ist ebenfalls möglich, die Wirkstoffe allein einer Sprühtrocknung oder Sprühkristallisation zu unterwerfen und anschließend in einem Sprühmischer mit den Perlglanzwachsen zu beaufschlagen. Hier wird üblicherweise ein Perlglanzwachs/Wachsgemisch in flüssiger Form, d.h. etwa 5 bis 10°C über dessen Schmelzpunkt, auf eine den Wirkstoff enthaltene Matrix aufgesprüht. Die den Wirkstoff enthaltende Matrix liegt als Festkörper vor und kann aus hydrophilen Wachsen, kristallisierenden Tensidzubereitungen oder den Wirkstoff bindenden Trägermaterialien, welche mit dem Wirkstoff kompatibel sind, bestehen. Man wählt vorzugsweise ein Gewichtsverhältnis von Perlglanzwachs : Wirkstoff von 10:1 bis 1:1, vorzugsweise 7:1 bis 2:1. Es werden Korngrößen von 3 bis 100, vorzugsweise 4 bis 50 und insbesondere 5 bis 25 µm erhalten. Die Sprühtrocknung bzw. Sprühkristallisation kann in einer bevorzugten Ausführungsform der Erfindung mit Hilfe eines Doppelrohres durchgeführt werden, wobei die Perlglanzwachse durch das äußere Rohr und die Wirkstoffe durch das Innenrohr eingeleitet werden. Auf diese Weise können die Wirkstoffe einen inneren Kem bilden, der von den Perglanzmitteln umgeben in einer kontinuierlichen Phase umgeben ist. Unter einer kontinuierlichen Phase wird bevorzugt eine homogene Phase verstanden, die auch über einen längeren Lagerzeitraum unverändert stabil bleibt.

### Kosmetische und/oder pharmazeutische Zubereitungen

Die Mittel der vorliegenden Erfindung dienen zur Herstellung von kosmetischen und/oder pharmazeutischen Zubereitungen, als da sind Haarshampoos, Mund- und Zahnpflegemittel, Haarlotionen, Schaumbäder, Duschbäder, Cremes, Gele, Lotionen, alkoholische und wäßrig/alkoholische Lösungen, Emulsionen, Wachs/Fett-Massen, Stiftpräparaten oder Salben. Diese erfindungsgemäßen Mittel können den Zubereitungen in Mengen von 0,01 bis 30, vorzugsweise 0,5 bis 20 und insbesondere 1 bis 10 Gew.-% - bezogen auf den Aktivsubstanzgehalt - enthalten sein. Als weitere Hilfs- und Zusatzstoffe können sie milde Tenside, Ölkörper, Emulgatoren, Überfettungsmittel, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe und dergleichen aufweisen. Eine Reihe oieser Hilfsstoffe sind schon in den vorherigen Kapiteln näher erläutert worden, so daß an dieser Stelle auf eine Wiederholung verzichtet wird.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, VinylpyrrolidonNinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperaturmethode.

### Beispiele

Die kosmetischen Zubereitungen, welche aus einer kontinuierlichen Phase und einem darin dispergierten Wirkstoff bestehen (Compound 1 bis 6), wurden durch gemeinsame Sprühkristallisation der unten aufgeführten Wirkstoffe mit den Perlglanzwachsgemischen hergestellt. Die Compounds 1 bis 6 setzen sich wie folgt zusammen:
1) Dipalmitoylethyl Hydroxyethylmonium Methosulfate : Cetearyl Alcohol : Ethylenglycoldistearat (25 : 20 : 55)
2) Uvasorb : Cetearyl Alcohol: Ethylenglycoldistearat (40 : 15 : 45)
3) Ceramide 3 : Distearylether : Cetearyl Alcohol (25 : 50 : 25)
4) Ceramide 3 : Ethylenglycoldistearate (25 : 75)
5) Milchsäure : Ethylenglycolmonostearat : Cetearyl Alcohol (25 : 50 : 25)
6) Alpha Tocophero I: Ethylenglycolmonostearat : Cetearyl Alcohol : Glycerinmonostearat (20 : 40 : 20 : 20)

Die nachfolgenden Tabellen geben einige ausgewählte Beispiele für kosmetische Zubereitungen an, in denen die erfindungsgemäßen Compounds eingesetzt (jeweils Beispiel 1) werden. Als Vergleich **(V1)** wurden Zubereitungen ohne diese Compounds hergestellt. Das Aussehen dieser Zubereitungen wurde beurteilt und die Lagerstabilität bei 20 °C und einem Zeitraum von 4 Wochen beurteilt (+ = stabil; - = instabil; separiert/sedimentiert, agglomeriert).

**Tabelle 1:**

| **Conditioning Shampoo - Mengenangaben in Gew.-% Aktivsubstanz -** | | | |
|---|---|---|---|
| **Zusammensetzung** | **V1** | **1** | **2** |
| Sodium laureth sulfate | 10 | 10 | 10 |
| Cocamidopropylbetaine | 3 | 3 | 3 |
| Dipalmitoylethyl Hydroxyethylmonium Methosulfate | 0,5 | - | - |
| Cetearyl Alcohol | 0,4 | 0,3 | 0,3 |
| Ethylenglycoldistearat | 1,1 | - | - |
| Compound 1 | - | 2 | 4 |
| Wasser | ad 100 | | |
| **Aussehen** | trüb/perlglänzend | trüb/perlglänzend | trüb/perlglänzend |
| **Lagerstabilität** | - | + | + |

**Tabelle 2:**

| **Shampoo mit öllöslichem UV Filter- Mengenangaben in Gew.-% Aktivsubstanz -** | | | |
|---|---|---|---|
| **Zusammensetzung** | **V1** | **1** | **2** |
| Sodium laureth sulfate | 10 | 10 | 10 |
| Cocomidopropylbetaine | 2 | 2 | 2 |
| Cocoglucoside | 2 | 2 | 2 |
| Uvasorb® HEB | 0,8 | - | - |
| Cetearyl Alcohol | 0,3 | - | - |
| Ethylenglycoldistearat | 0,9 | - | - |
| Compound 2 | - | 2 | 4 |
| Wasser | ad 100 | | |
| **Aussehen** | trüb/perlglänzend | trüb/perlglänzend | trüb/perlglänzend |
| **Lagerstabilität** | - | + | + |

**Tabelle 3:**

| **Pflege Shampoo mit Ceramide 3 - Mengenangaben in Gew.-% Aktivsubstanz -** | | | |
|---|---|---|---|
| **Zusammensetzung** | **V1** | **1** | **2** |
| Sodium laureth sulfate | 10 | 10 | 10 |
| Cocomidopropylbetaine | 4 | 4 | 4 |
| Cocoglucoside | 2 | 2 | 2 |
| D-Panthenol | 0,3 | 0,3 | 0,3 |
| Ceramide 3 | 0,5 | - | - |
| Distearylether | 1 | - | - |
| Cetearyl Alcohol | 1 | - | - |
| Compound 3 | - | 2,5 | 5 |
| Wasser | ad 100 | | |
| **Aussehen** | trüb/perlglänzend | trüb/perlglänzend | trüb/perlglänzend |
| **Lagerstabilität** | - | + | + |

**Tabelle 4:**

| **Spülmittel mit Ceramide 3 - Menge nangaben in Gew.-% Aktivsubstanz -** | | | |
|---|---|---|---|
| **Zusammensetzung** | **V1** | **1** | **2** |
| Sodium laureth sulfate | 15 | 15 | 15 |
| Cocomidopropylbetaine | 4 | 4 | 4 |
| Cocoglucoside | 6 | 6 | 6 |
| Aloe Vera | 0,1 | 0,1 | 0,1 |
| Ceramide 3 | 0,5 | - | - |
| Ethylenglycoldistearat | 1,5 | - | - |
| Compound 4 | - | 2 | 4 |
| Glycerin | 4 | 4 | 4 |
| Ethanol | 4 | 4 | 4 |
| Wasser | ad 100 | | |
| **Aussehen** | trüb/perlglänzend | trüb/perlglänzend | trüb/perlglänzend |
| **Lagerstabilität** | - | + | + |

**Tabelle 5:**

| **Pflege Shampoo mit Milchsäure - Mengenangaben in Gew.-% Aktivsubstanz -** | | | |
|---|---|---|---|
| **Zusammensetzung** | **V1** | **1** | **2** |
| Sodium laureth sulfate | 9 | 9 | 9 |
| Cocomidopropylbetaine | 3 | 3 | 3 |
| Cocoglucoside | 4 | 4 | 4 |
| D-Panthenol | 0,3 | 0,3 | 0,3 |
| Milchsäure | 0,5 | - | - |
| Ethylenglycoldistearat | 1 | - | - |
| Cetearyl Alcohol | 0,5 | - | - |
| Compound 5 | - | 2 | 4 |
| Wasser | ad 100 | | |
| **Aussehen** | trüb/perlglänzend | trüb/perlglänzend | trüb/perlglänzend |
| **Lagerstabilität** | - | + | + |

**Tabelle 6:**

| **Pflege Shampoo mit ViatminE - Mengenangaben in Gew.-% Aktivsubstanz -** | | | |
|---|---|---|---|
| **Zusammensetzung** | **V1** | **1** | **2** |
| Sodium laureth sulfate | 8 | 8 | 8 |
| Cocomidopropylbetaine | 3 | 3 | 3 |
| Cocoglucoside | 4 | 4 | 4 |
| D-Panthenol | 0,3 | 0,3 | 0,3 |
| Alpha Tocopherol | 1 | - | - |
| Ethylenglycoldistearat | 2 | - | - |
| Cetearyl Alcohol | 1 | - | - |
| Glycerinmonostearat | 1 | - | - |
| Compound 6 | - | 4 | 8 |
| Wasser | ad 100 | | |
| **Aussehen** | trüb/perlglänzend | trüb/perlglänzend | trüb/perlglänzend |
| **Lagerstabilität** | - | + | + |

## Patentansprüche

1. Kosmetische Zubereitungen bestehend aus einer kontinuierlichen Phase und darin dispergierten Wirkstoffen, **dadurch gekennzeichnet, dass** die kontinuierliche Phase aus Perlglanzwachsen besteht.

2. Mittel nach Anspruch 1, **dadurch gekennzeichnet, dass** sie Perlglanzwachse enthalten, die ausgewählt sind aus der Gruppe, die gebildet wird von Alkylenglycolestem, Fettsäurealkanolamiden, Partialglyceriden, Estem von mehrwertigen, gegebenenfalls hydroxysubstituierten Carbonsäuren mit Fettalkoholen mit 6 bis 22 Kohlenstoffatomen, Fettalkoholen, Fettketonen, Fettaldehyden, Fettethem und/oder Fettcarbonaten, die in Summe mindestens 24 Kohlenstoffatome aufweisen, Fettsäuren und Hydroxyfettsäuren mit 16 bis 30 Kohlenstoffatomen; sowie Ringöffnungsprodukten von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen.

3. Mittel nach den Ansprüchen 1 und/oder 2, **dadurch gekennzeichnet, dass** sie feste oder flüssige Perlglanzwachse enthalten.

4. Mittel nach mindestens einem der Ansprüche 1 bis 3, **dadurch gekennzeichnet, dass** sie Wirkstoffe enthalten, die ausgewählt sind aus der Gruppe, die gebildet wird von Tensiden, kosmetischen Ölen, Stabilisatoren, biogenen Wirkstoffen, Vitaminen, Deodorantien, Antitranspirantien, Antischuppenmitteln, UV-Lichtschutzfaktoren, Antioxidantien, Konservierungsmitteln, Insektenrepellentien, Selbstbräunem, Parfümölen, Aromastoffen und Farbstoffen.

5. Mittel nach mindestens einem der Ansprüche 1 bis 4, **dadurch gekennzeichnet, dass** die Wirkstoffe einen inneren Kem bilden, der von der kontinuierlichen Phase umhüllt ist.

6. Verfahren zur Herstellung von kosmetischen und/oder pharmazeutischen Zubereitungen, bei dem man die Wirkstoffe einer Sprühtrocknung oder Sprühkristallisation unterwirft und anschließend mit dem Perlglanzwachs beaufschlagt.

7. Verfahren zur Herstellung von kosmetischen und/oder pharmazeutischen Zubereitungen, bei dem man eine Mischung aus Perlglanzwachsen und Wirkstoffen einer gemeinsamen Sprühtrocknung oder Sprühkristallisation unterwirft.

8. Verfahren nach Anspruch 6, **dadurch gekennzeichnet, dass** man die Sprühtrocknung oder Sprühkristallisation mit einem Doppelrohr durchführt, bei dem die Perglanzwachse durch ein äußeres Rohr und die Wirkstoffe durch ein Innenrohr geleitet werden.

9. Verfahren nach mindestens einem der Ansprüche 6 bis 8, **dadurch gekennzeichnet, dass** man die Sprühtrocknung oder Sprühkristallisation unter vermindertem Druck durchführt.

10. Verwendung von Mitteln nach Anspruch 1 zur Herstellung von kosmetischen und/oder pharmazeutischen Zubereitungen.

## Claims

1. Cosmetic preparations consisting of a continuous phase and active principles dispersed therein, **characterized in that** the continuous phase consists of pearlizing waxes.

2. Preparations as claimed in claim 1, **characterized in that** they contain pearlizing waxes selected from the group consisting of alkylene glycol esters, fatty acid alkanolamides, partial glycerides, esters of polybasic, optionally hydroxysubstituted carboxylic acids with fatty alcohols containing 6 to 22 carbon atoms, fatty alcohols, fatty ketones, fatty aldehydes, fatty ethers and/or fatty carbonates containing in all at least 24 carbon atoms, fatty acids and hydroxyfatty acids containing 16 to 30 carbon atoms and ring-opening products of olefin epoxides containing 12 to 22 carbon atoms with fatty alcohols containing 12 to 22 carbon atoms and/or polyols containing 2 to 15 carbon atoms and 2 to 10 hydroxyl groups.

3. Preparations as claimed in claims 1 and/or 2, **characterized in that** they contain solid or liquid pearlizing waxes.

4. Preparations as claimed in at least one of claims 1 to 3, **characterized in that** they contain active principles selected from the group consisting of surfactants, cosmetic oils, stabilizers, biogenic agents, vitamins, deodorants, antiperspirants, antidandruff agents, UV protection factors, antioxidants, preservatives, insect repellents, self-tanning agents, perfume oils, aromas and dyes.

5. Preparations as claimed in at least one of claims 1 to 4, **characterized in that** the active principles form an inner core which is surrounded by the continuous phase.

6. A process for the production of cosmetic and/or pharmaceutical preparations in which the active principles are subjected to spray drying or spray crystallization and then charged with the pearlizing wax.

7. A process for the production of cosmetic and/or pharmaceutical preparations in which a mixture of pearlizing waxes and active principles is subjected to spray drying or spray crystallization.

8. A process as claimed in claim 6, **characterized in that** spray drying or spray crystallization is carried out using a double tube in which the pearlizing waxes are introduced through an outer tube and the active principles through an inner tube.

9. A process as claimed in at least one of claims 6 to 8, **characterized in that** spray drying or spray crystallization is carried out under reduced pressure.

10. The use of the preparations claimed in claim 1 for the production of cosmetic and/or pharmaceutical preparations.

## Revendications

1. Préparations cosmétiques qui consistent en une phase continue et en des principes actifs dispersés dans celle-ci,
**caractérisées en ce que**
la phase continue est formée de cires à éclat nacré.

2. Préparations selon la revendication 1,
**caractérisées en ce qu'**
elles renferment des cires à éclat nacré choisies dans le groupe formé des esters d'alkylèneglycol, des alkanolamides d'acide gras, des glycérides partiels, des esters d'acides carboxyliques plurifonctionnels, éventuellement substitués par un hydroxyle avec des alcools gras ayant de 6 à 22 atomes de carbone, des alcools gras, des cétones grasses, des aldéhydes gras, des éthers gras, et/ou des carbonates gras, qui possèdent globalement au moins 24 atomes de carbone, des acides gras et des acides gras hydroxylés ayant de 16 à 30 atomes de carbone, ainsi que des produits d'ouverture de cycle d'époxyde d'oléfine ayant de 12 à 22 atomes de carbone avec des alcools gras ayant de 12 à 22 atomes de carbone et/ou des polyols ayant de 2 à 15 atomes de carbone et de 2 à 10 groupes hydroxyle.

3. Préparations selon les revendications 1 et/ou 2,
**caractérisées en ce qu'**
elles renferment des cires à éclat nacré solides ou liquides.

4. Préparations selon au moins une des revendications 1 à 3,
**caractérisées en ce qu'**
elles renferment des principes actifs qui sont choisis dans le groupe formé des agents tensioactifs, des huiles cosmétiques, des agents stabilisants, des principes actifs biogènes, des vitamines, des agents désodorants, des agents antiperspirants, des agents antipelliculaires, des facteurs de protection contre la lumière UV, des agents antioxydants, des agents conservateurs, des répulsifs contre les insectes, des agents auto-bronzants, des essences de parfum, des substances odoriférantes et des colorants.

5. Préparations selon au moins l'une des revendications 1 à 4,
**caractérisées en ce que**
les principes actifs forment un noyau interne qui est enrobé par la phase continue.

6. Procédé de production de préparations cosmétiques et/ou pharmaceutiques,
selon lequel
on soumet les principes actifs à un séchage par pulvérisation ou à une cristallisation par pulvérisation et ensuite on met en contact avec la cire à éclat nacré.

7. Procédé de production de préparations cosmétiques et/ou pharmaceutiques,
selon lequel
on soumet un mélange à base de cires à éclat nacré et de principes actifs, à un séchage par pulvérisation ou à une cristallisation par pulvérisation conjointe.

8. Procédé selon la revendication 6,
**caractérisé en ce qu'**
on exécute le séchage par pulvérisation ou la cristallisation par pulvérisation avec un tube double dans lequel les cires à éclat nacré sont amenées par un tube extérieur et les principes actifs par un tube intérieur.

9. Procédé selon au moins une des revendications 6 à 8,
**caractérisé en ce qu'**
on exécute le séchage par pulvérisation ou la cristallisation par pulvérisation sous pression réduite.

10. Utilisation de préparations selon la revendication 1,
en vue de la production de produits cosmétiques et/ou pharmaceutiques.
